# EUROPÄISCHE PATENTSCHRIFT

(11) **EP 1 165 038 B1**
(45) Veröffentlichungstag und Bekanntmachung des Hinweises auf die Patenterteilung: **12.05.2004**
(21) Anmeldenummer: 00916982.2
(22) Anmeldetag: 21.03.2000
(51) Int. Cl.: A61K 7/48

(54) **MISTELEXTRAKTE IN HAUTKOSMETIKA**
MISTLETOE EXTRACTS FOR USE IN SKIN COSMETICS
EXTRAITS DE GUI S'UTILISANT EN COSMETIQUE DERMATOLOGIQUE

(30) Priorität: 30.03.1999 DE 19914366
(43) Veröffentlichungstag der Anmeldung: 02.01.2002
(73) Patentinhaber: Henkel Kommanditgesellschaft auf Aktien, 40589 Düsseldorf-Holthausen (DE)
(72) Erfinder: GERKE, Thomas, D-41468 Neuss (DE); SÄTTLER, Andrea, D-40225 Düsseldorf (DE)
(86) Internationale Anmeldenummer: PCT/EP2000/002463
(87) Internationale Veröffentlichungsnummer: WO 2000/059464

(56) Entgegenhaltungen:
- FR-A- 1 588 770
- US-A- 5 773 014
- DATABASE WPI Week 199814 Derwent Publications Ltd., London, GB; AN 1998-146057 XP002140515 "Cosmetic composition - comprises extract from mistletoe grown on trees of genus Eulalyptus, optionally with suitable base or carrier" & AU 33150 97 A (MILIOTIS), 12. Februar 1998 (1998-02-12) in der Anmeldung erwähnt
- CHEMICAL ABSTRACTS, vol. 67, no. 12, 18. September 1967 (1967-09-18) Columbus, Ohio, US; abstract no. 57244v, "Emulsion cosmetics decreasing the sensitivity of skin to cold" XP002140514 & PL 51 542 A (WARSZAWSKA FABRYKE MYDLA I KOSMETYKOW) 20. Juli 1966 (1966-07-20)
- P. W. JOLLER ET AL : "Stimulation of cytokine production via a special standardized mistletoe preparation in an in vitro human skin bioassay" ARZNEIMITTEL FORSCHUNG. DRUG RESEARCH., Bd. 46, Nr. 6, 1996, Seiten 649-653, XP002140513 EDITIO CANTOR. AULENDORF., DE ISSN: 0004-4172
- PATENT ABSTRACTS OF JAPAN vol. 1998, no. 6, 30. April 1998 (1998-04-30) & JP 10 029922 A (SHISEIDO), 3. Februar 1998 (1998-02-03) in der Anmeldung erwähnt
- PATENT ABSTRACTS OF JAPAN vol. 2000, no. 2, 29. Februar 2000 (2000-02-29) & JP 11 322569 A (LION ), 24. November 1999 (1999-11-24)

## Beschreibung

Die Erfindung betrifft die Verwendung von Mistelextrakten in kosmetischen Zusammensetzungen zur Tyrosinase-Hemmung. Zusammensetzungen dieser Art dienen u. a. der kosmetischen Aufhellung der Haut und der aufhellenden Behandlung von Sommersprossen und Leberflecken.

Für die Hautfarbe sind vor allem Melanine verantwortlich, braune bis schwarze Pigmente, die in bestimmten Zellen der Epidermis, sogenannten Melanocyten gebildet werden. Während Menschen verschiedener Hautfarbe über eine vergleichbare Melanocytenzahl verfügen, ergeben sich die Farbunterschiede durch unterschiedliche Verteilung und Konzentration des Melanins. Sommersprossen und Leberflecken resultieren aus einer erhöhten Melanin-Konzentration in entsprechenden Zellen und Gewebepartien. Chemisch gesehen handelt es sich bei Melanin um polymere, sehr komplexe Aggregate chinoider Substanzen, die sich vom Indol-Gerüst ableiten und unter der Einwirkung bestimmter Enzyme entstehen. Das Schlüsselenzym der Reaktionsfolge, Tyrosinase, das durch UV-Strahlen aktiviert wird, oxidiert Tyrosin in einer Reaktionskaskade zu Melanin. Bei erhöhter UV-Belastung wird vermehrt Melanin produziert. Melanine sind natürliche Lichtschutzfaktoren und übernehmen vor allem eine Schutzfunktion für die Zellkerne, indem sie die UV-Strahlung stark abschwächen.

Eine unerwünschte Pigmentierung wird mit sogenannten Depigmentierungsmitteln behandelt, die eine Hautbleichung und Aufhellung der Pigmentflecken bewirken. Während die Präparate in Europa ein eher geringes Verbraucherinteresse finden, haben sie in Ländern mit farbiger Bevölkerung, insbesondere in asiatischen Ländern ein wesentlich höheres Marktvolumen, da die Mehrzahl der asiatischen Frauen eine möglichst helle Hautfarbe als ästhetisch empfindet.

Eine Hautbleichung kann über einen Eingriff in einen oder mehrere der Regulationsmechanismen der Pigmentbildung erreicht werden, deren gemeinsames Ziel es ist, die Melanin-Synthese zu hemmen. Aus toxikologischen Gründen sind zahlreiche früher verwendete Wirkstoffe, wie z. B. Hydrochinonether, Resorcin und verschiedene Peroxide, die direkt eine Zerstörung der Melanocyten oder eine Reduktion bzw. Oxidation des Melanins bewirken, nicht mehr in Hautpflegemitteln zugelassen. Auch die Verwendung von Quecksilber- und Wismutsalzen, welche Tyrosinase und Dopa-Oxidase irreversibel hemmen, sind in den meisten Ländern unzulässig. Als häufigster Wirkstoff zur Hautbleichung wird noch immer Hydrochinon verwendet, welches leichter oxidierbar ist als Tyrosin und dadurch dessen Oxidation zum Melanin-Pigment verhindert. Hydrochinon gilt jedoch ebenfalls als toxikologisch bedenklich; hydrochinonhaltige Präparate dürfen daher nur unter Einschränkung in Kosmetika angewendet werden.

Als vielversprechender hat sich eine Hautaufhellung über Tyrosinase-Inhibitoren, wie z. B. Ascorbinsäure, erwiesen. Jüngste Bestrebungen der Kosmetikindustrie zunehmend "Naturmittel" einzusetzen, führten zu einem intensiven Screening von Pflanzenextrakten bezüglich ihrer Wirkungsweise (Lee, K. T.; Kim, B. J. ; Kim, J. H.; Heo, M. Y.; Kim, H. P. *International Journal of Cosmetic Science* **1997**, *19*, S. 291-298 und Kim, B. J. ; Kim J. H.; Kim, H. P.; Heo, M.Y., *idem,* 299-307). Die Verwendung tyrosinasehemmender Pflanzenextrakte in kosmetischen Zubereitungen wird u. a. in JP 10029922, US 5773014, JP 09286738, JP 10029928, JP 08301758 beschrieben. Die Verwendung von Mistelextrakten als "anti-ageing composition" und "post sun damage treatment" ist aus AU 9733150 bekannt.

Die derzeit zur Hautaufhellung eingesetzten Pflanzenextrakte werden den Ansprüchen nicht völlig gerecht. So tritt eine effektive tyrosinasehemmende Wirkung häufig erst bei höheren Konzentrationen der Extrakte ein und zahlreiche Pflanzenextrakte haben darüber hinaus unerwünschte toxische Nebenwirkungen. Eine Kombination verschiedener erwünschter Wirkstoffeigenschaften in einem einzigen Pflanzenextrakt ist nur für wenige Pflanzen bekannt.

Aufgabe war es daher, Pflanzenextrakte zu finden, die auch bei niedriger Dosierung Tyrosinase hemmen und darüber hinaus ggf. noch antioxidative Eigenschaften besitzen.

Überraschenderweise wurde nun gefunden, daß Mistelextrakt auch in relativ geringen Konzentrationen ein überaus effektiver Tyrosinase-Inhibitor mit antioxidativer Wirkung ist.

Gegenstand der Erfindung ist daher die Verwendung von Mistelextrakt zur Herstellung einer Zusammensetzung zur tyrosinasehemmenden, topischen Behandlung der Haut.

Mistelgewächse (Loranthaceae) kommen weltweit mit ca. 1100 Arten, vorzugsweise in den Tropen vor. *Viscum album*, der einheimische Vertreter, wächst als immergrüner Halbschmarotzer auf verschiedenen Bäumen Europas und Nordasiens (z. B. Eiche, Kastanie, Tanne, Kiefer, Linde, Pappel, Apfel). Seit altersher sind Misteln als Arznei- und "Zaubermittel" bekannt und enthalten neben dem stark toxischen Viscotoxin zahlreiche weitere Inhalts- und Wirkstoffe, so z. B. Acetylcholin, Cholin, Lektine, Flavonoide, Lignane, Inosite, Saponine und Phenylpropan-Derivate. Die Wirkstoff schwankungen bei Viscum-Arten sind vermutlich auf die verschiedenen Wirtspflanzen zurückzuführen, da zwischen Wirtspflanze und Mistel eine enge biochemische Abhängigkeit besteht. So fällt beispielsweise die Toxizität von *Viscum album* in folgender Reihenfolge der Wirtspflanzengruppen ab: a) Acer, Tilia, Juglans, Robinia, Populus; b) Betula, Rosa, Fraxinus; c) Pinus, Abies; d) Malus silvestris; und e) Malus domestica.

Im Sinne der Erfindung werden Mistelextrakte in Kosmetika zur Hemmung der Tyrosinase eingesetzt. Dies hat eine kosmetische Aufhellung der Haut zur Folge. Unter Hemmung im Sinne der Erfindung wird die Aktivitätsabnahme einer Tyrosinase in Anwesenheit des Mistel-Extraktes im *in*-*vitro*-Test verstanden (Definition der Enzymaktivität, vgl. Stichwort "Enzyme" in: CD Römpp Chemie Lexikon, Version 1.0, Stuttgart, New York: Georg Thieme Verlag 1995). Vermutlich handelt es sich hierbei um eine kompetitive Hemmung des Enzyms durch Polyphenol-Verbindungen.

Im Sinne der Erfindung können die Extrakte aus allen Teilen der Pflanze (Blätter, Blüten, Früchte, Samen, Zweige, Rinde, Wurzel) der beiden Gattungen Viscum und Loranthus der Familie Loranthaceae gewonnen werden. Bevorzugt sind Extrakte aus Mistelblättern der Gattung Viscum, zu der u. a. die Arten Viscum album, Viscum laxus und Viscum abietus zählen. In einer bevorzugten Variante der Erfindung wird der Extrakt aus Blättern von *Viscum album* gewonnen und ist in einer Menge von mindestens 0.001 - 10 Gew.-% der Gesamtzusammensetzung enthalten. Die Gewichtsangabe bezieht sich dabei auf den reinen Extrakt, der nach Abziehen des Lösungsmittels im Vakuum erhalten wird. Bevorzugt sind Blattextrakte von *Viscum album*, die durch Extraktion mit Wasser und / oder mindestens einem organischen Lösungsmittel gewonnen wurden. Die wäßrige Phase kann neutral, sauer oder basisch sein, wobei der pH-Wert mit Carbonsäuren. Mineralsäuren oder Basen eingestellt werden kann. Als organische Lösungsmittel geeignet sind beispielsweise Alkohole, Ether, Ketone, Ester sowie halogenierte und nichthalogenierte aliphatische und aromatische Kohlenwasserstoffe bzw. Mischungen der entsprechenden Lösungsmittel, die üblicherweise für Extraktionen eingesetzt werden (z. B.: Rydberg, J.; Musikas, C.; Choppin, G. R. *Principles and Practices of Solvent Extraction*, Dekker, New York, 1992). Bezüglich des Temperaturbereiches besteht keine prinzipielle Einschränkung, obgleich eine Extraktion bei Raumtemperatur bevorzugt sein kann.

Je nach Pflanzenherkunft und Extraktionsmittel oder -verfahren erhält man zwischen 5 und 50% Ausbeute. Die Tyrosinase-Hemmung wurde in Gegenwart von 0,01 bis 1,0 Vol.-% Mistelextrakt untersucht. Eine ausgeprägte Hemmwirkung bei sehr geringer Konzentration wurde bei Extrakten festgestellt, die durch Extraktion der Mistelteile mit Alkohol/Eisessig (Extrakte 1-3, Tabelle 1) oder mit Isooctan erhalten wurden (Extrakt 6, Tabelle 1).

In einer bevorzugten Ausführungsform der Erfindung wird ein Mistelextrakt verwendet, der dadurch gekennzeichnet ist, daß er durch Extraktion mit einem organischen Lösungsmittel aus der Gruppe der Alkohole mit 1 bis 4 C-Atomen gewonnen wurde. Hierzu gehören beispielsweise Methanol, Ethanol, Propanol, Isopropanol, 1-Butanol, 2-Butanol, *tert*-Butanol, 2-Methylpropan-1-ol, aber auch mehrwertige Alkohole wie z. B. Glycerin oder 1,2-Propandiol. Im Sinne der Erfindung können auch Alkoholgemische, Gemische der Alkohole mit anderen organischen Lösungsmitteln oder mit Wasser verwendet werden.

Ebenfalls bevorzugt ist die Verwendung eines Mistelextraktes, der dadurch gekennzeichnet ist, daß er durch Extraktion mit einem Gemisch aus einem Alkohol und einer Carbonsäure gewonnen wurde. Üblicherweise verwendete Alkohole sind Methanol. Ethanol, Propanol, Isopropanol, 1-Butanol, 2-Butanol, *tert*-Butanol, 2-Methylpropan-1-ol. aber auch mehrwertige Alkohole wie z. B. Glycerin oder 1,2-Propandiol. Als Carbonsäure können beispielsweise Ameisensäure, Essigsäure, Citronensäure, Äpfelsäure, Sorbinsäure. Benzoesäure und Azelainsäure eingesetzt werden, obgleich die Auswahl nicht hierauf beschränkt ist. Alkohol/Carbonsäure-Gemische mit 0,5 - 5,0 Mol% Carbonsäure sind im Sinne der Erfindung bevorzugt. Auch Gemische von Alkoholen und Mineralsäuren sind als Extraktionsmittel verwendbar.

Die Verwendung eines Mistelextraktes der dadurch gekennzeichnet ist, daß er durch Extraktion mit einem organischen Lösungsmittel aus der Gruppe der Kohlenwasserstoffe mit bis zu 8 C-Atomen gewonnen wurde, ist im Sinne der Erfindung ebenfalls bevorzugt. Besonders geeignet sind die gesättigten aliphatischen Lösungsmittel (n- und iso-Alkane sowie Cycloalkane), wie beispielsweise Isooctan, Heptan, Cyclohexan, Cyclopentan, Pentan, aber auch Hexan, obgleich letzteres wegen seiner Toxizität nur noch relativ wenig zum Einsatz kommt.

In einer weiteren bevorzugten Variante der Erfindung wird Mistelextrakt verwendet, der dadurch gekennzeichnet ist, daß er durch Extraktion mit einem organischen Lösungsmittel aus der Gruppe der Ketone mit bis zu 6 C-Atomen gewonnen wurde. Hierzu zählen acyclische und cyclische aliphatische Ketone. Die Verwendung von Aceton, Methylethylketon, Diisopropylketon und / oder Cyclohexanon kann besonders bevorzugt sein.

Im Sinne der Erfindung können auch Ketongemische, Gemische der Ketone mit anderen organischen Lösungsmitteln oder mit Wasser verwendet werden.

Die Verwendung von Mistelextrakten, die dadurch gekennzeichnet sind, daß sie zusätzlich antioxidativ wirken, sind im Sinne der Erfindung besonders geeignet. Als antioxidative Wirkung oder "antioxidatives Potential" im Sinne der Erfindung bezeichnet man radikalfangende Eigenschaften, die in Analogie zu DD 249 618 A3 mittels Chemilumineszenz gemessen wurden. Die antioxidativen Wirkstoffe im Mistelextrakt verhindern, daß freie Sauerstoff-Radikale die Lipid-Membranen der lebenden Zellen angreifen (Lipidperoxidation) oder gar zu irreversiblen Schäden der DNA führen. Neuere Forschungen haben gezeigt, daß auch die externe Anwendung von Antioxidantien den "oxidativen Stress" vermindern kann. Die Bemühungen der Kosmetik-Industrie haben die Entwicklung von Körperpflegemitteln gefördert, die als Präventivmaßnahme hochwirksame und insbesondere physiologisch unbedenkliche Antioxidantien enthalten, wobei in jüngster Zeit vermehrt Naturstoff-Extrakte zum Einsatz kommen. Für kosmetische Zwecke ist die Kombination einer tyrosinasehemmenden und antioxidativen Wirkung in Mistelextrakten besonders günstig, da eine verstärkte Pigmentierung der Haut nach UV-Belastung oft zusammen mit oxidativen Schädigungen auftritt. Eine Hautaufhellung durch Tyrosinasehemmung wird also von einer schonenden antioxidativen Prophylaxe/Behandlung begleitet. Eine tyrosinasehemmende und antioxidative Wirkung zeigen die Mistelextrakte (1) - (5), Tabelle 1 und 2, wobei das antioxidative Potential der Extrakte (4) und (5) besonders ausgeprägt ist.

Die Verwendung von Mistelextrakten, die einen Lektingehalt von weniger 0.01 Gew.-% aufweisen, kann bevorzugt sein. Lektine sind in allen lebenden Organismen weit verbreitete Eiweißstoffe, die sehr spezifisch Saccharide und Polysaccharide erkennen und binden, auch wenn diese in Lipid- oder Protein-gebundener Form vorliegen. Lektine würden nicht selten für Lebensmittelvergiftungen verantwortlich gemacht und zeichnen sich durch ihre Fähigkeit aus, Erythrocyten zu agglutinieren. Da man bisher wenig über die biologischen Funktionen, Wirkungsweise und Toxizität der Lektine weiß, kann es vorteilhaft sein, Extrakte von Misteln zu verwenden, deren Lektingehalt niedrig ist (Jaeggy, C.; Musielski, H.; Urech, K.; Schaller, G. *Arzneim*.-*Forsch*. **1995**, *45*, S. 905-909 und Park, W. B.; Ju, Y. J.; Han, S. K. *Arch. Pharmacal. Res*. **1998**, *21*, 429-435).

Ebenso kann die Verwendung von Extrakten bevorzugt sein, die einen Viscotoxingehalt von weniger 0.01 Gew.-% aufweisen. Viscotoxin gilt als das toxische Prinzip aus Misteln (Viscum album) und gehört zur Gruppe der Thionine, stark basische pflanzliche Polypeptide mit cytotoxischen Eigenschaften, die sich durch einen besonders hohen Cystein-Gehalt auszeichnen. Viscotoxin besteht aus fünf basischen Polypeptiden, von denen vier als homogene Verbindungen isoliert werden konnten. Ob die tumornekrotisierende Wirkung von Mistelextrakt auf Viscotoxin zurückgeht, ist bisher umstritten. Viscotoxin löst sich in Wasser und Methanol sehr leicht, schwer dagegen in Alkohol und Eisessig; in Ether, Chloroform, Benzol und Aceton ist es dagegen unlöslich. Beim Erwärmen wird es leicht zersetzt. Der Viscotoxingehalt läßt sich durch die Auswahl der Wirtspflanze (vide supra), Extraktionsmittel und -temperatur kontrollieren (Schaller, G.; Urech, K.; Giannattasio, M. *Phytother. Res*. **1996**, *10*, S. 473-477 und Jordan, E.; Wagner, H. *Arzneim.-Forsch* **1986**, *36*, S. 428-433).

Eine Verwendung von Mistelextrakten, die dadurch gekennzeichnet ist, daß es sich bei der tyrosinasehemmenden Behandlung der Haut um eine kosmetische Aufhellung der Haut handelt, ist besonders bevorzugt. Hierzu zählt auch die aufhellende Behandlung von Sommersprossen und / oder sogenannten Altershautflecken.

Depigmentierungsmittel werden auf die Gesichtshaut und / oder andere hyperpigmentierte Partien des Körpers aufgetragen. Üblicherweise werden sie als wäßrig-alkoholische Lösungen oder dünnflüssige bis cremeförmige O/W-Emulsionen formuliert, die vorzugsweise einen pH-Wert von 6-7 aufweisen, aber auch die Formulierung von W/O-Emulsionen kann im Sinne der Erfindung vorteilhaft sein. Je nach Anforderung ist jedoch auch eine Formulierung als Gel oder sogar als Badezusatz denkbar. Die zusätzliche Verwendung von Sonnenschutzfiltern kann ggf. vorteilhaft sein. Neben Wasser und physiologisch geeigneten Lösungsmitteln können u. a. pflegende Bestandteile, Öle, Wachse, Fette, rückfettende Substanzen, Verdickungsmittel, Emulgatoren und Duftstoffe enthalten sein. Für geeignete Formulierungs-Grundlagen sei an dieser Stelle auf die in der Kosmetik üblichen Rezepturen verwiesen (K. Schrader, *Grundlagen und Rezepturen der Kosmetika*, 2. Aufl., Hüthig Buch Verlag, Heidelberg 1989, Seite 424-437, 442-451, 456-465, 609-618 und W. Umbach, *Kosmetik*, 2. Aufl., Georg Thieme Verlag, Stuttgart 1995. Seite 132-135,341, 115-116).

### Extraktionsvorschrift

Typischerweise wurden zerkleinerte oder gepulverte Mistelteile oder -blätter (ca. 40.0g) in eine 250 mL-Soxhlethülse eingewogen, die Hülse mit Watte verschlossen und 24h mit 1500 mL Extraktionsmittel (dies kann auch ein Gemisch von Lösungsmitteln sein) in der Siedehitze extrahiert. Nach dem Abkühlen wurde der Extrakt ggf. neutralisiert und im Vakuum eingeengt und dann, um eine bessere Lagerstabilität zu gewährleisten, in ca. 100 mL Ethanol aufgenommen. Die Extrakte wurden dunkel, in verschlossenen Flaschen aufbewahrt. Die Ausbeuten lagen je nach Herkunft der Pflanze und nach Extraktionsmittel zwischen 5 und 50% bezogen auf die Trockenmasse der eingesetzten Pflanzenteile. Das Lösungsmittel (Ethanol) wurde erst vor Messung der Tyrosinase-Hemmung bzw. des antioxidativen Potentials im Vakuum abdestilliert.

### Tyrosinase-Hemmung

Die Aktivität der Pilz-Tyrosinase (Sigma) wurde in Abhängigkeit verschiedener Mistelextrakt-Konzentrationen (0,01 - 1,0 Vol.-%) anhand der enzymatischen Umsetzung von L-DOPA zu Dopachrom bestimmt. Das Absorptionsmaximum des Dopachroms (rotbraun) liegt bei λ = 475 nm. Der lineare Anstieg der Absorption (A) des Dopachroms pro Zeiteinheit (t) ist ein Maß für die Aktivität der Tyrosinase (ΔA/Δt).

Die Aktivität der Tyrosinase in Abwesenheit des Mistelextraktes, (ΔA₁/Δt₁) diente als Referenz und wurde auf 100% gesetzt. Unter analogen Bedingungen wurde die Tyrosinase-Aktivität in Gegenwart eines Mistelextraktes (ΔA₂/Δt₂) bestimmt. Die Hemmwirkung oder Minderung der Tyrosinase-Aktivität entspricht dann: 100% - (ΔA₂/Δt₂ )/( ΔA₁/Δt₁ )%. Jede Messung wurde zweifach, in parallelen Ansätzen durchgeführt. Die Schwankung der Methode liegt bei ca. ± 10%.

### Verwendete Chemikalien

| | |
|---|---|
| L-3,4-Dihydroxyphenylalanin (L-DOPA) | (Sigma) |
| KH₂PO₄ | (J. T. Baker) |
| Tyrosinase, 50.000 units | (Sigma) |
| KOH | |

### Benötigte Lösungen

- 50 mM KH₂PO₄-Puffer
   Einstellung auf pH = 6,5 mit 1M KOH (in H₂O bidest.)
- 2,5 mM L-DOPA (in H₂O bidest.)
- 340 U/mL Tyrosinase-Stammlösung (in kaltem KH₂PO₄-Puffer, pH = 6,5)

### Reaktionscocktail

10 mL KH₂PO₄-Puffer
10 mL L-DOPA
9 mL H₂O bidest.

Der Reaktionscocktail wird ebenso wie die Tyrosinase-Stammlösung erst vor Versuchsbeginn hergestellt. Die Tyrosinase-Stammlösung muß unbedingt kühl gelagert werden (Kühlschrank oder Eisbad). Die L-DOPA Lösungen sollten dunkel und in gut verschlossenen Gefäßen unter Sauerstoffausschluß aufbewahrt werden. Bei evtl. Grauverfärbung (Oxidation durch Luftsauerstoff) muß die Lösung frisch angesetzt werden.

### Mistelextraktlösungen

Von den Mistelextrakten wurden 10%ige Stammlösungen (Vol.%) im Reaktionscocktail hergestellt. Um homogene Lösungen zu erhalten, wurden die Proben kurz in ein Ultraschallbad gehalten und dann ggf. vor Verwendung nochmals zentrifugiert. Ein entsprechendes Aliquot der Mistelextrakt-Stammlösung wurde dann zur Probenlösung (Testsystem) gegeben. Die üblichen Mistelextrakt-Konzentrationen im Testsystem (in Vol.%) betragen 0,01%, 0,05%, 0,1%, 0,25%, 0,5% und 1 % Mistelextrakt.

### Testsystem (Probenvolumen 1mL) und Reaktionsdurchführung

33 µL Tyrosinase-Stammlösung
Aliquot der Mistelextrakt-Stammlösung
Ad 1000 µL Reaktionscocktail

Als Referenz dient eine Probe ohne Mistelextrakt. Sämtliche Proben wurden vor Meßbeginn am Vibrofix gut durchmischt. Der pH-Wert wurde kontrolliert und ggf. auf pH = 6,5 eingestellt. Als erstes wurde die Referenzprobe vermessen. Die Messung erfolgte mit einem Uvikon-860-Photometer der Fa. Kontron.
Die Absorption des Dopachroms wurde am Absorptionsmaximum bei λ = 475 nm über einen Zeitraum von 5 min. bei 25°C detektiert, wobei das Meßintervall 20-30 sec betrug.

### Ergebnis:

In Tabelle 1 ist die Restaktivität der Tyrosinase in Abhängigkeit der Mistelextrakt-Konzentration zusammengestellt. Die Tyrosinase-Aktivität der Referenz wurde auf 100% gesetzt. Von den Proben (1) - (5) wurde zusätzlich das antioxidative Potential bestimmt (Tabelle 2).

**Tabelle 1**

| **Nr.** | **Pflanzenextrakte** | **Konzentrationen im Test [Vol.-%]** | | | | | |
|---|---|---|---|---|---|---|---|
| | | 0,01 | 0,05 | 0,1 | 0,25 | 0,5 | 1,0 |

| | | **Restaktivität derTyrosinase in %** | | | | | |
|---|---|---|---|---|---|---|---|
| (1) | Mistel (Ungarn) Ethanol:Eisessig 90:10^{a)} | 102 | --- | 101 | 38 | 11 | --- |
| (2) | Mistel (Türkei) Ethanol:Eisessig 90:10^{a)} | 106 | 104 | ---- | ---- | 22 | --- |
| (3) | Mistel (Ungarn) t-Butanol:Eisessig 90:10^{a)} | 107 | 106 | 29 | --- | --- | --- |
| (4) | Mistelblätter, zerkleinert Ethanol | 100 | --- | 80 | --- | --- | 60 |
| (5) | Mistelblätter, zerkleinert Ethanol:Eisessig 90:10^{a)} | 100 | --- | 85 | --- | --- | 26 |
| | | | | | | | |
| (6) | Mistelextrakt Isooctan | 111 | --- | 103 | --- | 45 | 25 |
| (7) | Mistel (Türkei) Ethanol | 100 | --- | 96 | --- | --- | 71 |
| (9) | Mistel (Türkei) Isooctan | 101 | --- | 98 | --- | --- | 63 |
| (10) | Mistel (Ungarn) Aceton | 102 | --- | 94 | --- | --- | 78 |

| | | | | | | | |
|---|---|---|---|---|---|---|---|
| a) Angabe bezieht sich auf Volumenteile | | | | | | | |

### Bewertung

Hervorragende tyrosinasehemmende Eigenschaften bei niedriger Mistelextrakt-Konzentration resultieren bei Extraktion der Misteln mit einem Gemisch aus Alkohol und Eisessig (Extrakte 1-3). Auch bei Extraktion mit Kohlenwasserstoffen, zeigt der resultierende Extrakt eine deutliche tyrosinasehemmende Wirkung (Extrakt 6), während Ethanol- und Acetonextrakte in höheren Konzentrationen eingesetzt werden müssen, um eine effektivere Inhibition der Tyrosinase zu erreichen.

### Messung des antioxidativen Potentials (AOP)

Das antioxidative Potential (AOP) charakterisiert die "radikalfangenden" Eigenschaften einer Substanz oder eines Substanzgemisches.

Eine detaillierte Beschreibung der Methode und Apparatur findet sich in **DD 249 618 A3**. Die Methode beruht auf der durch Sauerstoffradikale induzierten Photochemilumineszenz von Luminol (3-Aminophthalsäurehydrazid) in Abwesenheit und in Gegenwart bestimmter Radikalfänger (Antioxidantien). Die Photochemilumineszenz in Abwesenheit von Antioxidantien dient als Leerwert. In Gegenwart von Radikalfängern wird eine Abnahme der Chemilumineszenz des Luminols beobachtet. Zur Kalibrierung des Systems wird eine definierte Menge Vitamin E zugegeben und die Chemilumineszenz in Gegenwart dieses Radikalfängers bestimmt. Dieser Wert dient als Referenz und wird als **1 Vitamin-E-Einheit** bezeichnet. Die durch die Prüfsubstanz hervorgerufene Lumineszenzabnahme wird in Relation zur Lumineszenzabnahme durch Vitamin E gesetzt und in Vitamin-E-Einheiten ausgedrückt. In Relation zu Vitamin E läßt sich somit das antioxidative Potential zahlreicher Substanzen und Substanzgemische bestimmen.

### Durchführung der AOP-Messungen

### 1. Prinzipieller Aufbau des Meßsystems

Die Apparatur besteht prinzipiell aus 2 Komponenten, einem Radikal-Generator und einem Detektorsystem.
Mittels einer UV-Strahlungsquelle werden im wäßrigen Medium durch Reduktion oder Anregung von Sauerstoff-Molekülen Superoxidradikal-Anionen oder Singulettsauerstoff erzeugt. Die entstandenen freien Radikale reagieren mit Luminol, dessen Chemilumineszenz über das Detektorsystem quantitativ erfaßt wird.

### 2. Benötigte Chemikalien

- Luminol, HPLC-grade (Fa. Baker)
- Methanol, HPLC-grade (Fa. Baker)
- Ethanol, HPLC-grade (Fa. Baker)
- Na₂CO₃
- Na₂EDTA·2H₂O
- NaOH
- α-Tocopherol (Fa. Merck)

### 3. Benötigte Geräte

Photochem, Fa. FAT, Berlin
Software: Poplab 2.0
Vibrofix
Übliches Labormaterial:
Spritzenfilter, 0,2 µm, Fa. Millipore

### 4.Versuchsdurchführung

### 4.1 Stammlösungen

### (A) ACL-Puffer-Stammlösung (ACL = Antioxidative Capacity of Lipid soluble substances)

Zur Herstellung des ACL-Puffers (pH = 11) wurden 10,6 g Na₂CO₃ (M=106,0 g/mol) und 0,032 g Na₂EDTA·2H₂O (M=372,24 g/mol) eingewogen und mit Milli-Q-Wasser auf 1 Liter aufgefüllt. Die Pufferlösung wurde über ein 0,45 µm Spritzenfilter filtriert.

### (B) Methanolischer ACL - Puffer

Es wurden 50 mL der ACL-Pufferstammlösung (A) mit 950 mL Methanol (HPLC-grade) versetzt und solange gerührt, bis eine klare Lösung resultierte.

### (C) Luminol-Lösung

Zur Detektion wurde eine 1 mM Luminol-Lösung in 0,1 M Natronlauge verwendet. Die Lösung muß lichtgeschützt und gekühlt aufbewahrt werden.

### 4.2 Meßlösungen

### (D) Lösung zur Leerwertbestimmung

In ein verschließbares Autosamplergläschen (4 mL) wurden 2,0 mL des vorbereiteten klaren methanolischen ACL-Puffers pipettiert. Hierzu wurden ca. 100 µL der Luminollösung und soviel wasserfreies unvergälltes Ethanol gegeben, daß die Summe der Volumina von Ethanol und Luminollösung 0,5 mL ergab. Die Lösung wurde am Vibrofix wenige Sekunden lang homogenisiert und unverzüglich am Photochem vermessen (vgl. 4.4).

### (E) Proben-Lösung

In einem 10 mL-Meßkolben wurde die analytisch genau eingewogene Menge (500 ± 20 mg) der zu analysierenden Probe mit Methanol bis zur Marke aufgefüllt. Die Lösung wurde 10 min mit Ultraschall behandelt und anschließend filtriert.

### (F) Meßlösung

In ein verschließbares 4 mL-Autosamplergläschen wurden 2,0 mL methanolischen ACL-Puffer (vgl. B) pipettiert. Hierzu gab man dasselbe Volumen Luminollösung, das auch für die Leerwertbestimmung verwendet wurde, eine definierte Menge der filtrierten Probenlösung (E) und soviel wasserfreies unvergälltes Ethanol, daß die Summe der Volumina von Luminollösung, Probenlösung und Ethanol 0,5 mL ergab. Die Lösung wurde am Vibrofix wenige Sekunden lang homogenisiert und unverzüglich am Photochem vermessen.

### 4.3 Kalibrierung

Die Kalibierung erfolgte mit einem Vitamin-E-Standard. In einem 50 mL Meßkolben wurde eine analytisch genau eingewogene Menge d,l-α-Tocopherol (80 ± 5 mg) mit Methanol bis zur Marke aufgefüllt. Diese Lösung wurde mit Ethanol so verdünnt, daß sich ein Gehalt von 12 mg/L ergab.

Zur Kalibrierung wurden Lösungen (je 2,5 mL) mit jeweils 30, 50 und 100 µL Vitamin-E-Standardlösung hergestellt. Hierzu kombinierte man je 2 mL methanolische ACL-Pufferlösung mit dem gleichen Volumen Luminollösung, das auch für die Leerwertbestimmung verwendet wurde, und gab jeweils 30, 50 und 100 µL Vitamin-E-Standardlösung und soviel wasserfreies unvergälltes Ethanol zu, daß die Summe der Volumina von Luminollösung, Vitamin-E-Standardlösung und Ethanol 0,5 mL ergab. Die Messung erfolgte wie unter 4.4. beschrieben.

### 4.4 Messung der Chemolumineszenz

Nach Einschalten des Gerätes und nach jeder Pause wurde die Chemilumineszenz der Leerwert-Lösung (vgl. D) bestimmt. Wenn die Meßwerte stabil blieben, konnte die eigentliche Messung gestartet werden. Die Meßdauer betrug pro Probe 180 Sekunden. Der Leerwert (Integral unter der Kurve) wurde von der Software automatisch bei den nachfolgenden Meßungen berücksichtigt.
Anschließend wurde das System auf Vitamin-E-Standards (vgl. 4.3) kalibriert, d. h. die Chemilumineszenz der drei Vitamin-E-Standards bestimmt. Dann wurde die Chemilumineszenz der Meßlösungen (F) der zu untersuchenden Proben bestimmt. Die Inhibition der Chemilumineszenz ergab sich aus dem Integral des Leerwertes minus dem Integral der Meßlösung normiert auf das Integral des Leerwertes.

### 5. Berechnung des Analysenergebnisses

Die für die Meßlösungen ermittelten Kurven wurden in Relation zu den Kalibrationskurven für Vitamin E gesetzt. Die für eine Substanzprobe spezifische Inhibition der Chemilumineszenz wurde in Vitamin-E-Äquivalente pro g Probe ausgedrückt (mg Vit. E /g Probe) und als sogenanntes *Antioxidatives Potential* (AOP) bezeichnet.

### Testergebnisse (AOP)

Es wurden zahlreiche Mistelextrakte unterschiedlicher Herkunft bezüglich ihres antioxidativen Potentials untersucht.

Exemplarisch sind in Tabelle 2 die Ergebnisse für einige Extrakte zusammengefaßt, die in den erfindungsgemäßen Formulierungsbeispielen enthalten sind und ein besonders ausgeprägtes AOP zeigen.

**Tabelle 2**

| ***Mistelextrakte*/*Extraktionsmittel*** | ***AOP (Vitamin E-Einheiten)*** |
|---|---|
| (1) Mistel (Ungarn)/ Ethanol:Eisesseig 90:10 | 6,7 |
| (2) Mistel (Türkei)/ Ethanol:Eisesseig 90:10 | 7,2 |
| (3) Mistel (Ungarn)/ t-Butanol:Eisessig 90:10 | 6,2 |
| (4) Mistelblätter, zerkleinert/ Ethanol | 26 |
| (5) Mistelblätter, zerkleinert/ Ethanol:Eisessig 90:10 | 15,5 |

### Bewertung:

Die Mistelextrakte (1)-(5) zeigen neben der tyrosinasehemmenden Wirkung zusätzlich antioxidative Wirkung. Gewisse Schwankungen in den AOP-Werten sind vermutlich auf die unterschiedlichen Wirtspflanzen und/oder Herkunftsländer zurückzuführen. Bei allen Extrakten ist die antioxidative Wirkung jedoch signifikant höher als die des Vitamin E, wobei die Extrakte (4) und (5) besonders geeignet sind. Folglich ist die Verwendung von Mistelextrakt als tyrosinasehemmender Wirkstoff und zusätzlich als antioxidative Prophylaxe in Kosmetika außerordentlich vorteilhaft.

### Erfindungsgemäße Formulierungsbeispiele

Alle nachfolgenden Mengenangaben sind Gew.-% der handelsüblichen Substanzen oder Substanzgemische bezogen auf die Gesamtmenge der Zusammensetzung.

### 1. Wäßriges Liposomengel

4,0% Phospholipid (z. B. Sternprime® N10)
6,0% Ethanol
0,2% Konservierungsmittel (z. B. Phenonip®)
0,5% vernetztes Polyacrylat (z. B. Carbopol® ETD 2020)
5,0% Mistelextrakt (1)
ad 100% H₂O(dest.)

Das Lecithin wurde oberhalb seiner Hauptphasenumwandlungstemperatur durch Einspritzen einer ethanolischen Stammlösung (40 Gew.-%) in Wasser (ca. 50-60 °C) dispergiert. Nach Abkühlen der Dispersion auf ca. 40 °C wurde der Mistelextrakt und die kalte Polyacrylat-Quellung zugegeben. Anschließend wurde mit 10 % -iger NaOH-Lösung auf pH=7 neutralisiert.
Gegebenenfalls können weitere wasser- oder öllösliche Wirkstoffe (Vitamin E, Panthenol, Sonnenschutzfilter, etc.) auf bekannte Weise eingearbeitet werden.

### 2. Öl-in-Wasser PIT-Creme

8,0% Dicaprylylether (z. B. Cetiol® OE)
8,0% Dioctylcyclohexan (z. B. Cetiol® S)
4,0% Cetearylakohol (z. B. Lanette® O)
3,0% Ceteareth-20 (z. B. Eumulgin® B2)
1,5% Glycerylpalmitat (z. B. Monomuls® 60-35C)
0,5% Silikonöl (z. B. Baysilon® M 350)
1,0% Vitamin E
5,0% Glycerin
0,3% Konservierungsmittel (z. B. Phenonip®)
3,0 % Mistelextrakt (3)
ad 100% H₂O(dest.)

Zuerst wurde ein Emulsionskonzentrat nach dem Phaseninversionsverfahren (PIT) hergestellt (T. Förster in "Surfactants in Cosmetics", Hrsg.: M. M. Rieger und L. D. Rhein, Marcel Dekker, New York 1997, Bd. 2). Die Phaseninversionstemperatur liegt zwischen 70 und 84 °C. Das heiße Emulsionskonzentrat wurde abgekühlt und der Mistelextrakt bei 40 °C in Form einer wäßrigen Lösung zugegeben.

### 3. Wasser-in-Öl Emulsion

8,0% Dicaprylylether (z. B. Cetiol® OE) 8,0% Decyloleat (z. B. Cetiol® V)
3,5% hydriertes Rizinusöl-Ethoxylat (z. B. Dehymuls® HRE 7)
0,7% MgSO₄·7H₂O (z. B. Veegum®)
1,0% Vitamin E
5,0% Glycerin
0,3% Konservierungsmittel (z. B. Phenonip®)
7,0 % Mistelextrakt (6)
ad 100% H₂O(dest.)

Die Ölbestandteile wurden zusammen mit dem Emulgator auf 40 °C erwärmt und die wäßrige Lösung der weiteren Inhaltsstoffe unter Rühren zugegeben.

### 4. Gesichtswasser mit aufhellender Wirkung

50% Ethanol, 95%ig
1,0% Propylenglykol-8EO-monococoat
2,0% Ethylhexylsalicylat
0,50 Aluminiumnitrat
0,05% Parfümöl
10,0 % Mistelextrakt (4)
ad 100% H₂O(dest.)

### Anhang

### Verzeichnis der Warenzeichen der verwendeten Rohstoffe

1) Sternprime® N10
   Sojalecithin, desodoriert, standardisiert
   Hersteller: Stern Lecithin & Soja
2) Phenonip®:
   INCI: Phenoxyethanol, Methylparaben, Ethylparaben, Propylparaben, Butylparaben
   Hersteller: Nipa Laboratorien GmbH
3) Carbopol® ETD 2020
   INCI: Acrylates/C₁₀-C₃₀ alkyl acrylates cross polymer
   Hersteller: Goodrich
4) Cetiol® OE
   INCI: Dicaprylyl ether
   Hersteller: Henkel KGaA
5) Cetiol® S
   INCI: Dioctylcyclohexane
   Hersteller: Henkel KGaA
6) Lanette® O
   INCI: Cetearyl alcohol
   Hersteller: Henkel KGaA
7) Eumulgin® B2
   INCI: Cetheareth-20
   Hersteller: Henkel KGaA
8) Monomuls® 60-35C
   INCI: Hydrogenated palm glycerides
   Hersteller: Henkel KGaA
9) Baysilon® M 350
   INCI: Dimethicone
   Hersteller: Bayer AG
10) Cetiol® V
   INCI: Decyl oleate
   Hersteller: Henkel KGaA
11) Dehymuls® HRE 7
   INCI: PEG-7 hydrogenated castor oil
   Hersteller: Henkel KGaA
12) Veegum®
   INCI: Magnesium aluminum silicate
   Hersteller: Vanderbilt

## Patentansprüche

1. Verwendung einer kosmetischen Zusammensetzung, die Mistelextrakt enthält, zur tyrosinasehemmenden topischen Behandlung der Haut.

2. Verwendung gemäß Anspruch 1, **dadurch gekennzeichnet, daß** die Zusammensetzung zusätzlich der antioxidativen topischen Behandlung der Haut dient.

3. Verwendung gemäß Anspruch 1 oder 2, **dadurch gekennzeichnet, daß** der Extrakt aus Blättern von *Viscum album* gewonnen wurde und in einer Menge von mindestens 0.001 - 10 Gew.-% der Zusammensetzung enthalten ist.

4. Verwendung gemäß einem der Ansprüche 1 bis 3, **dadurch gekennzeichnet, daß** der Extrakt aus Blättern von *Viscum album* durch Extraktion mit Wasser und/oder mindestens einem organischen Lösungsmittel gewonnen wurde.

5. Verwendung gemäß Anspruch 4, **dadurch gekennzeichnet, daß** der Mistelextrakt durch Extraktion mit einem organischen Lösungsmittel aus der Gruppe der Alkohole mit 1 bis 4 C-Atomen gewonnen wurde.

6. Verwendung gemäß Anspruch 4, **dadurch gekennzeichnet, daß** der Mistelextrakt durch Extraktion mit einem Gemisch aus einem Alkohol und einer Carbonsäure gewonnen wurde.

7. Verwendung gemäß Anspruch 4, **dadurch gekennzeichnet, daß** der Mistelextrakt durch Extraktion mit einem organischen Lösungsmittel aus der Gruppe der Kohlenwasserstoffe mit bis zu 8 C-Atomen gewonnen wurde.

8. Verwendung gemäß Anspruch 4, **dadurch gekennzeichnet, daß** der Mistelextrakt durch Extraktion mit einem organischen Lösungsmittel aus der Gruppe der Ketone mit bis zu 6 C-Atomen gewonnen wurde.

9. Verwendung gemäß einem der Ansprüche 1 bis 8, **dadurch gekennzeichnet, daß** es sich bei der tyrosinasehemmenden Behandlung der Haut um eine kosmetische Aufhellung der Haut handelt.

## Claims

1. The use of a cosmetic composition containing mistletoe extract for the tyrosinase-inhibiting topical treatment of the skin.

2. The use claimed in claim 1, **characterized in that** the composition is additionally used for the antioxidative topical treatment of the skin.

3. The use claimed in claim 1 or 2, **characterized in that** the extract was obtained from leaves of *Viscum album* and is present in a quantity of at least 0.001 to 10% by weight of the composition.

4. The use claimed in any of claims 1 to 3, **characterized in that** the extract was obtained from leaves of Viscum album by extraction with water and/or at least one organic solvent.

5. The use claimed in claim 4, **characterized in that** the mistletoe extract was obtained by extraction with an organic solvent from the group of C₁₋₄ alcohols.

6. The use claimed in claim 4, **characterized in that** the mistletoe extract was obtained by extraction with a mixture of an alcohol and a carboxylic acid.

7. The use claimed in claim 4, **characterized in that** mistletoe extract was obtained by extraction with an organic solvent from the group of hydrocarbons containing up to 8 carbon atoms.

8. The use claimed in claim 4, **characterized in that** mistletoe extract was obtained by extraction with an organic solvent from the group of ketones containing up to 6 carbon atoms.

9. The use claimed in any of claims 1 to 8, **characterized in that** the tyrosinase-inhibiting treatment of the skin is a cosmetic lightening of the skin.

## Revendications

1. Utilisation d'une composition cosmétique qui contient de l'extrait de gui pour le traitement topique de la peau, inhibant la tyrosinase.

2. Utilisation selon la revendication 1, **caractérisée en ce que** la composition sert en outre au traitement topique antioxydant de la peau.

3. Utilisation selon la revendication 1 ou 2, **caractérisée en ce qu'**on obtient l'extrait à partir de feuilles de *Viscum album* et la composition contient l'extrait en une quantité d'au moins 0,001 à 10 % en poids.

4. Utilisation selon l'une quelconque des revendications 1 à 3, **caractérisée en ce qu'**on obtient l'extrait à partir de feuilles de *Viscum album* par extraction dans de l'eau et/ou dans au moins un solvant organique.

5. Utilisation selon la revendication 4, **caractérisée en ce qu'**on obtient l'extrait de gui par extraction dans un solvant organique choisi parmi le groupe des alcools contenant de 1 à 4 atomes de carbone.

6. Utilisation selon la revendication 4, **caractérisée en ce qu'**on obtient l'extrait de gui par extraction dans un mélange d'un alcool et d'un acide carboxylique.

7. Utilisation selon la revendication 4, **caractérisée en ce qu'**on obtient l'extrait de gui par extraction dans un solvant organique choisi parmi le groupe des hydrocarbures contenant jusqu'à 8 atomes de carbone.

8. Utilisation selon la revendication 4, **caractérisée en ce qu'**on obtient l'extrait de gui par extraction dans un solvant organique choisi parmi le groupe des cétones contenant jusqu'à 6 atomes de carbone.

9. Utilisation selon l'une quelconque des revendications 1 à 8, **caractérisée en ce que**, quant au traitement de la peau inhibant la tyrosinase, il s'agit d'un éclaircissement cosmétique de la peau.
